(19) Europäisches Patentamt
European Patent Office
Office européen des brevets

(11) Veröffentlichungsnummer: **0 161 436**
**B1**

(12) # EUROPÄISCHE PATENTSCHRIFT

(45) Veröffentlichungstag der Patentschrift: **02.09.87**

(21) Anmeldenummer: **85103548.5**

(22) Anmeldetag: **26.03.85**

(51) Int. Cl.⁴: **C 12 Q 1/28**, C 07 D 233/64, C 07 D 403/04, C 07 D 455/04, C 07 D 405/04

(54) **Neue Redox-Indikatoren.**

(30) Priorität: **31.03.84 DE 3411997**

(43) Veröffentlichungstag der Anmeldung: **21.11.85 Patentblatt 85/47**

(45) Bekanntmachung des Hinweises auf die Patenterteilung: **02.09.87 Patentblatt 87/36**

(84) Benannte Vertragsstaaten: **AT BE CH DE FR GB IT LI LU NL SE**

(56) Entgegenhaltungen:
**EP-A-0 030 684**
**EP-A-0 068 356**
**EP-A-0 068 438**
**EP-A-0 071 730**
**EP-A-0 122 641**
**DE-A-1 648 840**
**DE-A-1 917 996**
**DE-A-1 917 997**

**RESEARCH DISCLOSURE, Nr. 160, August 1977, Seiten 19 -24, no. 16034, Homewell, Havant, Hampshire, GB; "Compositions for the detection of H2O2"**

(73) Patentinhaber: **Boehringer Mannheim GmbH, Sandhofer Strasse 116, D-6800 Mannheim 31 (DE)**

(72) Erfinder: **Deneke, Ulfert, Dr.rer.nat., Birkenweg 5, D-6149 Rimbach- Zotzenbach (DE)**
Erfinder: **Güthlein, Werner, Dr.rer.nat., Im Sennteich 31, D-6800 Mannheim 24 (DE)**
Erfinder: **Kuhr, Manfred, Dr.rer.nat., Werthmannweg 23, D-6800 Mannheim 31 (DE)**
Erfinder: **Merdes, Hartmut, Dr.rer.nat., Schusterstrasse 11, D-6900 Heidelberg 1 (DE)**
Erfinder: **Murawski, Hans- Rüdiger, Dr.rer.nat, Carl- Lepper- Strasse 10, D-6840 Lampertheim (DE)**
Erfinder: **Wielinger, Hans, Dr.phil., Im Langgewann 7, D-6940 Weinheim (DE)**

**EP 0 161 436 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Reaktion von Wasserstoffperoxid mit Oxidationsindikatoren unter Katalyse von Peroxidase oder perixidatisch wirksamen Substanzen spielt in der analytischen Chemie eine besondere Rolle, weil sie außer dem Nachweis von Wasserstoffperoxid und Peroxidase auch die Bestimmung einer Reihe von Stoffen gestattet, die mit Sauerstoff und einer Reihe von Stoffen unter Bildung von Wasserstoffperoxid reagieren. Im folgenden seien einige dieser Stoffe als Beispiele aufgezählt und in Klammern die entsprechenden Oxidasen erwähnt:

Glucose (Glucoseoxidase), Galactose (Galactoseoxidase), L-Aminosäuren (L-Aminosäureoxidase), Cholesterin (Cholesterinoxidase), Harnsäure (Uricase), Sarkosin (Sarkosinoxidase) Glycerin (Glycerinoxidase), Pyruvat (Pyruvatoxidase).

Als Nachweisreaktion für Peroxidasen kommt die Methode insbesondere für die Bestimmung von Hämoglobin infrage.

Es sind dies vor allem Reaktionen, die in der medizinischen Diagnostik und in der Lebensmittelchemie von großer Bedeutung sind.

Die Nachweisreaktionen werden entweder in der Küvette oder mit Hilfe von Trockenreagenzträgern durchgeführt. Die Quantifizierung erfolgt dabei mit Photometern über eine Transmissionsmessung, mit Remissionsphotometern über Remissionsmessung oder mit Hilfe von Vergleichsfarben durch visuellen Vergleich.

Der Einsatz von Trockenreagenzträgern, d.h. saugfähigen oder quellfähigen Trägern, die mit den Reagenzien imprägniert sind bzw. in die die Reagenzien über andere Schritte eingearbeitet sind und auf denen nach Befeuchten mit dem Substrat die Nachweisreaktion abläuft, hat in jüngster Zeit immer mehr an Bedeutung zugenommen. Diese Hilfsmittel ermöglichen durch eine einfache Handhabung, bei gleichzeitig großer Zeitersparnis eine entscheidende Rationalisierung der entsprechenden Analysen. Die Forderung Trockenreagenzien zu entwickeln, bei denen mit unverdünnten Proben gearbeitet werden kann, stellt den Entwickler hinsichtlich der Auswahl des einzusetzenden Indikators bzw. Indikatorsystems vor das Problem, daß das Serum oder Plasma (im folgenden als Serum bezeichnet) die Nachweisreaktionen stark stört. Diese Störungen machen sich vor allem dann bemerkbar, wenn es notwendig ist, die Substrate bzw. Enzymaktivitäten über gekoppelte Reaktionsschritte nachzuweisen. Hier seien als Beispiel für Substrate die Nachweise von Creatinin und Harnsäure sowie als Beispiel für die Aktivitätsbestimmungen von Enzymen die Bestimmungen von Creatinkinase, Glutamat-Oxalacetat-Transaminase (GOT) und Glutamat-Pyruvat-Transaminase (GPT) erwähnt.

In der Literatur sind zahlreiche Verbindungen bekannt, die als Indikatoren für den Nachweis von Wasserstoffperoxid mit Peroxidase als Katalysator eingesetzt werden können. Solche Indikatoren sind: Benzidin und Benzidin-Derivate, verschiedene Phenole, Polyphenole wie z. B. Guajakharz, Leukofarbstoffe wie z. B. Leukomalachitgrün, Dichlorphenolindophenol, Aminocarbazole, Triarylimidazole, 2,2'-Azino-di-[3-äthyl-benzthiazolsulfonsäure-(6)] sowie Farbstoffe die als Kupplungsprodukt der oxidativen Kupplung von Aminoantipyrin oder verwandten Stoffen mit Phenolen, Naphtholen, Anilinderivaten und anderen Kupplungskomponenten entstehen.

Beim Nachweis von $H_2O_2$ in unverdünnten Serumproben weisen die vorstehenden bekannten Indikatoren mehr oder weniger starke Störungen durch Reaktion mit anderen Serumbestandteilen auf, die eine höhere oder meistens geringere Konzentration des nachzuweisenden Substrates vortäuschen. Relativ wenig gestört werden einige Triarylimidazole, wie sie in der DE-A 27 35 690 beschrieben sind. Die beschriebenen Imidazole sind nur im sauren pH-Bereich stabil und werden wie Experimente zeigten, beim Überführen in einen schwachsauren bis schwach alkalischen pH-Bereich, wie er bei fast allen enzymatischen Reaktionen notwendig ist, also dann, wenn sie als freie Base vorliegen, spontan vom Luftsauerstoff oxidiert. Eine Verarbeitung zu funktionsfähigen Trockenreagenzien mit diesen Indikatoren ist deshalb nür möglich, wenn sie in ein Schutzkolloid, wie z. B. Gelatine, eingebettet werden. Dies ist jedoch nur in speziellen Fällen durchführbar.

Die Aufgabenstellung der vorliegenden Erfindung war es daher, Farbstoffbildner für die Nachweisreaktion von Wasserstoffperoxid bzw. peroxidatisch wirksame Substanzen zu finden, die mit den im Serum enthaltenen Störsubstanzen nicht reagieren, im schwachsauren bis alkalischen Bereich von Luftsauerstoff nicht spontan oxidiert werden und somit sowohl in Küvettentests als auch in allen für Trockenreagenzträger brauchbaren Matrices verwendbar sind.

Es wurde überraschenderweise gefunden, daß Verbindungen der allgemeinen Formel I den oben gestellten Anforderungen entsprechen.

Gegenstand der Erfindung sind daher Imidazolderivate der Formel I

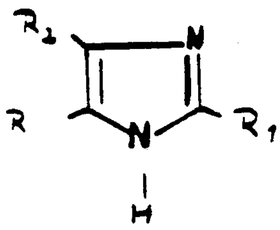

(I)

in der

R Wasserstoff, Tetrahydrofuranyl, Cycloalkyl oder Alkyl, das durch Hydroxy, Alkoxy, einen Schwefelsäure-, Phosphonsäure- oder Carbonsäurerest sowie eine Phenylgruppe substituiert sein kann,

und

$R_1$ und $R_2$, die gleich oder verschieden sein können, einen Julolidin-Rest, einen Tetrahydrochinolin-Rest, der gegebenenfalls am Stickstoffatom eine Alkylgruppe tragen kann, die wiederum durch einen Schwefelsäure-, Phosphonsäure- oder Carbonsäure-Rest substituiert sein kann,

oder einen Rest

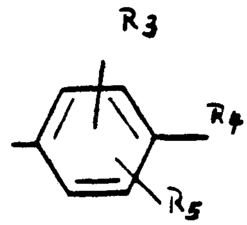

bedeuten,

in dem $R_4$ eine Hydroxy-, Amino-, eine mono- oder dialkylierte Aminogruppe, wobei die Alkylreste einen Schwefelsäure-, Phosphonsäure- oder Carbonsäure-Rest tragen können,

und

$R_3$ und $R_5$, die gleich oder verschieden sein können, Wasserstoff, Alkyl oder Alkoxy das gegebenenfalls mit einer Carboxylgruppe substituiert sein kann, darstellen,

mit der Maßgabe, daß $R_1$ und $R_2$ nicht gleichzeitig Julolidin- oder Tetrahydrochinolin sein dürfen und mmindestens ein $R_4$ der Substituenten $R_1$ und $R_2$ eine Hydroxygruppe sein muß,

sowie deren Salze.

Die Indikatoren gemäß allgemeiner Formel I lassen sich in alle bekannten Nachweissysteme einarbeiten.

Alkyl in den Substituenten $R_1$, $R_2$, $R_3$, $R_4$ und $R_5$ bedeutet Reste mit 1 - 6, vorzugsweise 1 - 4 Kohlenstoffatomen. Bevorzugt sind die Methyl-, Ethyl-, Butyl- und tert. Butyl-Gruppen.

Alkoxy in den Substituenten R, $R_3$ und $R_5$ bedeuten Reste mit 1 - 6, vorzugsweise 4 Kohlenstoffatomen. Bevorzugt ist die Methoxy- und Ethoxy-Gruppe.

Cycloalkyl in dem Substituenten R bedeutet einen Rest mit 3 - 8 Kohlenstoffatomen, bevorzugt ist der Cyclopropyl-, Cyclopentyl-, Cyclohexyl- und Cycloheptyl-Rest.

Die Schwefelsäure-, Phosphonsäure- und Carbonsäure-Reste, mit denen vorwiegend Alkyl-Gruppen substituiert sind, dienen insbesondere zur Löslichkeitsverbesserung.

Es lassen sich mit den Indikatoren Reagenzien herstellen, die in der Küvette vermessen werden. Dazu wird der Indikator zusammen mit Peroxidase, dem/den für den jeweiligen Parameternachweis notwendigen Enzym/en, anderen Reagenzien, dem Puffersystem, gegebenenfalls Netzmittel und anderen Hilfsstoffen lyophilisiert, als Pulver vermischt oder zu Tabletten verpreßt. Die so gewonnene Reagenzmischung wird vor Gebrauch in Wasser gelöst und so die Reagenzlösung bereitet. Nach Zugabe der Probe (Substratlösung, Enzymlösung, Serum oder Plasma) wird die eentstandene Farbe am Photometer vermessen und über den molaren Extinktionskoeffizienten und die zugesetzten Reagenz- bzw. Probevolumina die jeweilige Konzentration bzw. Enzymaktivität berechnet. Es sind sowohl kinetische als auch Endpunkts-Messungen möglich.

Ebenso können saugfähige Reagenzträger, wie Papiere, Vliese etc., mit den Indikatoren zusammen mit Peroxidase, dem/den für den jeweiligen Parameternachweis notwendigen Reagenzien bzw. anderen Enzymen, dem Puffersystem, gegebenenfalls Netzmitteln und anderen Hilfsstoffen imprägniert werden. Dazu kann man eine oder mehrere Imprägnierlösungen in Form von wässrigen oder organischen oder gemischten Lösungen herstellen, je nach dem, wie sich die Reagenzien oder Hilfsstoffe lösen. Mit diesen Lösungen werden Träger imprägniert oder besprüht. Anschließend wird getrocknet. Die so gewonnenen Reagenztrüger können als Schnelldiagnostika zur direkten Bestimmung von Inhaltsstoffen aus z. B. Körperflüssigkeiten eingesetzt werden. Dabei wird die Körperflüssigkeit direkt auf den Reagenzträger gebracht oder dieser in diese

eingetaucht. Durch Vergleichen der entstandenen Farbe mit Vergleichsfarben ist eine semiquantitative Bestimmung möglich. Über remissionsphotometrische Verfahren kann quantitativ ausgewertet werden. Es kann auch durch Eluieren der, wie vorstehend beschrieben, auf ein Papier oder Vlies imprägnierten Reagenzien mit Wasser oder Puffer eine Reagenzlösung hergestellt werden, mit der man wie oben beschrieben, Substrate oder Enzyme in einer Küvette am Photometer bestimmt (vgl. DE-A 2 301 999).

Eine weitere Möglichkeit der Verwendung der erfindungsgemäßen Indikatoren ist deren Einsatz in Reagenzfilmen zur quantitativen Bestimmung von Enzymen oder Substraten mittels eines Remissionsphotometers. Dabei wird der Indikator gemeinsam mit den anderen notwendigen Reagenzien und Hilfsstoffen, z. B. gemäß Verfahren entsprechend DE-C 1 598 153 oder DE-A 2 910 134 zu Reagenzfilmen verarbeitet.

Weiter hat es sich gezeigt, daß die erfindungsgemäßen Substanzen auch mit Stabilisatoren, wie sie in DE-C 2 716 060 beschrieben sind, erfolgreich kombiniert werden können. Diese Stabilisatoren (1-Aryl-semicarbazide) führen dazu, daß fertige Tests gegen Lichteinfluß unempfindlich werden und daß mit größeren Mengen die Funktionskurven der remissionsphotometrischen Messungen moduliert werden können.

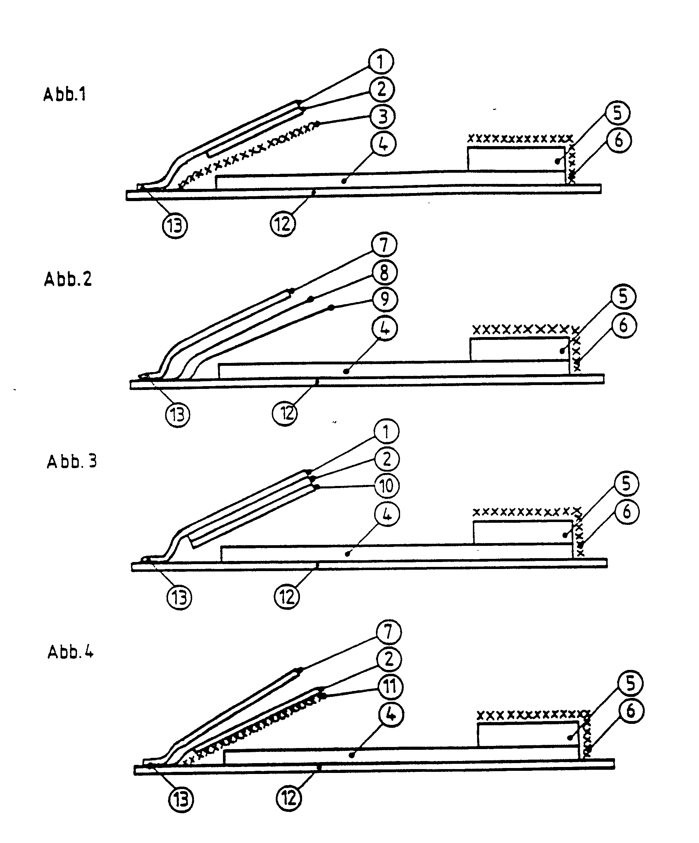

Die erfindungsgemäßen Indikatoren können, wie gesagt, in alle üblichen Reagenzträger, d. h. saugfähige Träger, wie Filterpapier, Vliese oder quell- bzw. saugfähige Reagenzfilme eingearbeitet werden (DE-C 15 98 153, DE-A 29 10 134, DE-A 32 47 608). Da sie vorzugsweise jedoch zum Nachweis von Enzymen und Substraten in Serum Verwendung finden sollen, sind in den Abbildungen 1 - 4 im Querschnitt eine Reihe von Vorrichtungen dargestellt, die gemäß DE-A 30 29 579 einerseits aus Vollblut das für den Test erforderliche Serum bzw. Plasma abtrennen und andererseits aufgrund eines speziell gestalteten Aufbaus der Reagenz- und Hilfstoffschichten eine Temperierung, Vorreaktion und gezieltes Starten der Hauptreaktion erlauben.

Im einzelnen sind die Vorrichtungen wie folgt zusammengesetzt:.

Abb. 1

Auf einer inerten Trägerfolie 12 ist ein aus Glasfasern bestehendes Vlies 4 befestigt, das einerseits zum Transport des Serums und andererseits zur Trennung von Serum und Erythrozyten dient. Dieses Vlies 4 teilweise überdeckend ist eine weitere aus Glasfasern bestehende Abtrennzone 5 mittels eines Fixiernetzes 6 befestigt. Auf dieses Fixiernetz 6 wird Vollblut aufgetragen, das in Abtrennzone 5 bzw. Vlies 4 in Serum und Erythrozyten getrennt wird, wobei letztere festgehalten werden, so daß in den linken Bereich des Vlieses 4 nur Serum übertritt. Seitlich von Vlies 4 sind über eine Klebestelle 13 ein dünnes Gewebe 3 sowie eine aus einem durchsichtigen Kunststoff bestehende Trägerfolie 1 befestigt. Unter der Trägerfolie 1 ist wiederum eine Reagenzzone 2 befestigt, die entweder aus einem quellfähigen oder saugfähigen Film besteht und in dem die für die Reaktion notwendigen Reagenzien eingearbeitet sind. Ein Teil der Reagenzien, insbesondere die für eine Vorreaktion notwendigen können bereits in Vlies enthalten sein. Durch Druck auf die Trägerfolie 1 wird die Reaktion gestartet, nachdem das Serum das Vlies 4 vollständig gefüllt hat, welches durch den Druckkontakt durch das Gewebe 3 in die Reagenzzone 2 eindringt und diese gleichmäßig durchfeuchtet. Sollte zusätzlicher Luftsauerstoff für die Reaktion notwendig sein, kann nach Durchfeuchtung der Reagenzzone die Vorrichtung wieder auseinandergetrennt werden. Die Reaktion wird anhand der Verfärbung in der Reagenzzone 2 durch die Trägerfolie 1 hindurch beobachtet.

Abb. 2

Der Aufbau der zur Gewinnung des Serums dienenden Zonen entspricht Abb. 1. Um eine Separierung der Reagenzien, die gegebenenfalls nicht miteinander lagerstabil sind, zu gewährleisten, sind zwei Reagenzpapiere 8 und 9 vorgesehen, die zusammen mit der schützenden Deckfolie 7 über die Klebestelle 13 mit der Trägerfolie 12 verbunden sind. Wiederum kann nach Sättigung des Vlieses 4 mit Serum durch Druck auf die Deckfolie 7 ein Flüssigkeitskontakt der Reagenzpapiere mit dem Serum bewirkt werden, der eine Vermischung des Serums und der Reagenzien in den Reagenzpapieren 8 und 9 bewirkt. Die Reaktion findet statt. Sie kann durch die Deckfolie 7 verfolgt werden.

Abb. 3

Diese Vorrichtung entspricht im Grundaufbau wiederum der Abb. 1, jedoch ist statt des Gewebes 3 ein optisch weißer Hintergrund 10 vorgesehen. Dieser optisch weiße Hintergrund 10 ist mit Bariumsulfat, Titandioxid oder ähnlichen stark reflektierenden Substanzen durchsetzt und besteht üblicherweise aus einem Kunststoff- oder Gelatine-Film. Durch 10 wird bewirkt, daß einerseits das zur Beobachtung der Reaktion eingestrahlte Licht vollständig remitiert wird und andererseits eventuelle Verfärbungen des Vlieses 4 nicht sichtbar werden können.

Abb. 4

entspricht bezüglich des Serumgewinnungsteils wiederum Abb. 1. Die Reagenzzone 2, die in diesem Fall aus einem Reagenzfilm besteht, ist in diesem Fall auf die eine Seite eines multifilen Gewebes 11 aufgetragen, welches einerseits zur Stabilisierung des Reagenzfilms dient und andererseits die Benetzung durch das Serum und den Zutritt von Luftsauerstoff fördert. Das Gewebe 11 und die lose aufgelegte Deckfolie 7 sind wiederum mit einer Klebestelle 13 an der Trägerfolie 12 befestigt. Durch Druck auf die Deckfolie 7 wird der Kontakt zwischen dem in Vlies 4 befindlichen Serum und der Reagenzzone 2 hergestellt und die Reaktion gestartet.

Es besteht natürlich auch die Möglichkeit die erfindungsgemäßen Substanzen in Gelatinematrices, gemäß DE-A 2 735 690 zusammen mit den für die entsprechende Nachweisreaktion benötigten Reagenzien und Hiifsstoffen einzuarbeiten.

Zusammenfassend ist festzustellen, daß die erfindungsgemäßen Verbindungen der allgemeinen Formel I in allen Testsystemen einsetzbar sind, mit deren Hilfe direkt oder nach vorangeschalteten Reaktionen

Wasserstoffperoxid oder peroxidatisch wirksame Substanzen nachgewiesen werden können.

Die erfindungsgemäßen Substanzen der Formel I werden in der Weise hergestellt, daß man in an sich bekannter Weise entweder

a) ein α-Diketon der Formel II

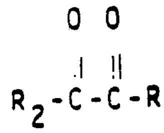

(II)

in der

$R_2$ und R die gleiche Bedeutung wie in Formel I besitzen, mit einem Aldehyd der Formel III

$O=CH-R_1$ (III)

in dem

$R_1$ die gleiche Bedeutung wie in der Formel I besitzt, mit Ammoniak in saurer Lösung kondensiert oder

b) ein α-Ketoxim der Formel IIa

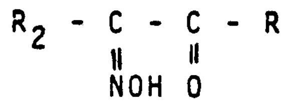

(IIa)

in der

$R_2$ und R die gleiche Bedeutung wie in Formel I besitzen, mit einem Aldehyd der Formel III und Ammoniak zu einer Verbindung der Formel Ia

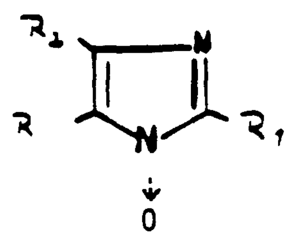

(Ia)

umsetzt und diese Verbindung in an sich bekannter Weise reduziert,

oder

c) ein Acylbromid der Formel IV

$R_2$-CO-CHR Br (IV)

in der

$R_2$ und R die gleiche Bedeutung wie in Formel I besitzen, mit einem Amidin der Formel V

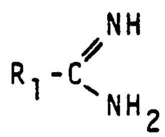

(V)

in der

$R_1$ die gleiche Bedeutung wie in der Formel I besitzt, in alkalischem Medium umsetzt

und anschließend, falls erforderlich, die erhaltenen Verbindungen in Verbindungen der Formel I überführt sowie entsprechende Imidazolbasen in Salze oder die Salze in die freien Basen umwandelt.

Die Überführung der nach dem Verfahren a) bis c) erhaltenen Verbindungen in Verbindungen der Formel I geschieht z. B. durch katalytiche Hydrierung (Nitro → Amin) Abspaltung von Schutzgruppen durch Hydrierung mit Pd/C (Benzyloxy → Hydroxy), Hydrierung von Heterocyclen (Furanyl → Tetrahydrofuranyl), Spaltung von Heterocyclen durch Hydrierung (Tetrahydrofuranyl → 4-Hydroxybutyl), reduktive Alkylierung von Aminen (Aminen → Dialkyl).

Durch Umsetzung von 4-Amino-Verbindungen der Formel I mit Halogen-methan- bzw. 2-ethan-carbonsäure,-Sulfonsäuren sowie -Phosphonsäuren bzw. ihren Salzen in DMF erhält man entsprechende N-alkylaminomethan- bzw. 2-ethan-Säure-reste.

Die Reduktion von N-oxiden der Formel Ia geschieht mit Zink/Essigsäure oder katalytisch erregtem Wasserstoff.

Die Synthese der Indikatoren gemäß allgemeiner Formel I erfolgt nach B.RADZIZEWSKI in Form ihrer Variante nach D. DAVIDSON (s. Org. Chem. 2, 319 (1937). In einigen Fällen wird anstelle des α-Diketons nach Lettau Chem. 10, 431 (1970), 11, 10 (1971) vorteilhafter das entsprechende α-Ketoxim verwendet. Die bei der Kondensation mit Aldehyd und Ammoniumacetat in Eisessig entstehenden Imidazol-N-oxide lassen sich relativ

leicht und mit guten Ausbeuten in die gewünschten Imidazole überführen. Als weiterer Syntheseweg dient die Umsetzung von Amidinen mit substituierten α-Phenacylbromiden, ferner die Ammonolyse von α-Acyloxyketonen (erhalten durch Umsetzung von α-Bromketonen mit Alkalisalzen von Carbonsäuren in DMF) mit Ammoniumacetat in Eisessig. Die Einwirkung gasförmiger Chlorwasserstoffsäure auf ein Gemisch von α-Aminonitril und Aldehyd bzw. die entsprechende Benzylidenverbindung führt unter Ringschluß zu den einschlägigen Imidazolen. Schließlich wird die katalytische Hydrierung von heterocyclischen Resten in diarylheterocyclisch-substituierten Imidazolen zur Herstellung von entsprechended perhydrierten Heterocyclen und offenkettigen Verbindungen herangezogen. Bei der reduktiven Methylierung von 4-Aminoarylimidazolen mit Formaldehyd und katalytisch erregtem Wasserstoff wird die NH-Gruppe am Imidazolring überraschenderweise nicht alkyliert, es entsteht vielmehr die entsprechende N-Dimethylaminoverbindung. Durch Überführung der neuen Imidazolbasen in Salze, z. B. Hydrochloride, Methansulfonate etc. erhält man in Wasser und Alkoholen lösliche, weitgehend stabile Indikatoren.

Bevorzugt im Sinne der Erfindung sind die folgenden Verbindungen:

1.) 2-(3,5-Dimethoxy-4-hydroxyphenyl)-4-(5)-(4-dimethylaminophenyl)-5-(4)-n-butyl-(1 H)-imidazol-hydrochlorid

2.) 2-(3,5-Dimethoxy-4-hydroxyphenyl)-4-(5)-(4-dimethylaminophenyl)-5-(4)-tert.butyl-(1 H)-imidazol-hydrochlorid

3.) 2-(3,5-Dimethoxy-4-hydroxyphenyl)-4-(5)-(4-dimethylaminophenyl)-5-(4)-(4-methoxybutyl)-(1 H)-imidazol-hydrochlorid

4.) 2-(3,5-Dimethoxy-4-hydroxyphenyl)-4-(5)-(4-dimethylaminophenyl)-5-(4)-cyclohexyl-(1 H)-imidazol-hydrochlorid

5.) 2-(3,5-Dimethoxy-4-hydroxyphenyl)-4-(5)-(4-dimethylaminophenyl)-5-(4)-benzyl-(1 H)-imidazol-hydrochlorid

6.) 2-(3,5-Dimethoxy-4-hydroxyphenyl)-4-(5)-(4-dimethylaminophenyl)-(1 H)-imidazolyl-5-(4)-methansulfonsäure

7.) 2-(3,5-Dimethoxy-4-hydroxyphenyl)-4-(5)-(4-dimethylaminophenyl)-(1 H)-imidazolyl-5-(4)-methanphosphonsäure

8.) 2-(3,5-Dimethoxy-4-hydroxyphenyl)-4-(5)-(4-dimethylaminophenyl)-(1 H)-imidazolyl-5-(4)-essigsäure

9.) 4-(5)-(3,5-Dimethoxy-4-hydroxyphenyl)-5-(4)-methyl-(1 H)-imidazolyl-2-(phenyl-4-N-methylamino-N-methan- bzw. N-ethan-2-sulfonsäure)

10.) 4-(5)-(3,5-Dimethoxy-4-hydroxyphenyl)-5-(4)-methyl-(1 H)-imidazolyl-2-(phenyl-4-N-methylamino-N-methan-, bzw. N-ethan-2-phosphonsäure)

11.) 4-(5)-(3,5-Dimethoxy-4-hydroxyphenyl)-5-(4)-methyl-(1 H)-imidazolyl-2-(phenyl-4-N-methylamino-essigsäure)

12.) 4-(5)-(3,5-Dimethoxy-4-hydroxyphenyl)-5-(4)-methyl-(1 H)-imidazolyl-2-(1,2,3,4-tetrahydrochinolino-6-methan-, bzw. ethan-2-sulfonsäure)

13.) 4-(5)-(3,5-Dimethoxy-4-hydroxyphenyl)-5-(4)-methyl-(1 H)-imidazolyl-2-(1,2,3,4-tetrahydrochinolino-6-methan-, bzw. ethan-2-phosphonsäure)

14.) 4-(5)-(3,5-Dimethoxy-4-hydroxyphenyl)-5-(4)-methyl-(1 H)-imidazolyl-2-(1,2,3,4-tetrahydrochinolino-6-essigsäure)

15.) 2-(3,5-Dimethoxy-4-hydroxyphenyl)-5-(4)-methyl-(1 H)-imidazolyl-4-(5)-(phenyl-4-N-methylamino-N-methan-, bzw. ethan-2-sulfonsäure)

16.) 2-(3,5-Dimethoxy-4-hydroxyphenyl)-5-(4)-methyl-(1 H)-imidazolyl-4-(5)-(phenyl-4-N-methylamino-N-methan-, bzw. N-ethan-2-phosphonsäure)

17.) 2-(3,5-Dimethoxy-4-hydroxyphenyl)-5-(4)-methyl-(1 H)-imidazolyl-4-(5)-(phenyl-4-N-methylaminoessigsäure)

18.) 2-(3,5-Di-tert-butyl-4-hydroxyphenyl)-5-(4)-methyl-(1 H)-imidazolyl-4-(5)-(phenyl-4-N-methylamino-N-methan-, bzw. ethan-2-sulfonsäure)

19.) 2-(3,5-Di-tert-butyl-4-hydroxyphenyl)-5-(4)-methyl-(1 H)-imidazolyl-4-(5)-(phenyl-4-N-methylamino-N-methan-, bzw. ethan-2-phosphonsäure)

20.) 2-(3,5-Di-tert-butyl-4-hydroxyphenyl)-5-(4)-methyl-(1 H)-imidazolyl-4-(5)-(phenyl-4-N-methylaminoessigsäure)

21.) 4-(5)-(4-Dimethylaminophenyl)-5-(4)-methyl-(1 H)-imidazolyl-2-(5[2-hydroxy-3-methoxy-phenoxyessigsäure])

An den nachfolgenden Beispielen soll die Erfindung näher erläutert werden.

Die Extinktionswerte bzw. die Extinktionskoeffizienten der beispielhaft offenbarten Verbindungen wurden nach der Methode gemäß Beispiel 10 ermittelt.

In den obengenannten Beispielen sowie in den nachfolgenden Beispielen bedeuten in den Verbindungsbezeichnungen die Klammer-Ausdrücke (4) bzw. (5) die jeweilige Substituentenposition der tautomeren Form der Imidazole der Formel I.

**Bespiel 1**

2-(3,5-Dimethoxy-4-hydroxyphenyl)-4-(5)-(4-dimethylaminophenyl)-5-(4) methyl-(1H)-imidazol-hydrochlorid

Eine Mischung von 57,4 g (0,5 Mol) 1-(4-Dimethylaminophenyl)-1,2-propandion, 60 g (0,33 Mol) 3,5-Dimethoxy-4-hydroxy-benzaldehyd (Syringaaldehyd) und 231 g (3 Mol) Ammoniumacetat werden in 1 l Eisessig im Ölbad unter Rühren und Argonatmosphäre 5 h am Rückfluß erhitzt. Danach kühlt man auf 15° C und tropft die Reaktionslösung auf 3,5 l 7 N Ammoniak *)

*) bei schwer absaugbaren Produkten nimmt man in Chloroform auf. Schuttelt mit Wasser durch, trocknet über Natriumsulfat und entfernt das Lösungsmittel im Vakuum

anschließend Saugt man den entstandenen Kristallbrei scharf ab und wäscht den Filterkuchen mit 3 x 150 ml Wasser. Nach Trocknen des Rohproduktes über Kaliumhydroxid führt man die Base in Methanol unter Zugabe von ca. 5 N etherischer Chlorwasserstoffsäure in das Hydrochlorid über. Nach Umkristallisieren aus einem geeigneten Lösungsmittel, z. B. Eisessig/Wasser (10/1) erhält man 101,4 g (71 % dTh) der Titelverbindung, farblose Kristalle, Fp. 185/219° C (Zers.), λmax 680 nm (ε = 25.500)

In analoger Weise erhält man durch Umsetzung der entsprechenden 1,2-Diketone und einschlägigen Aldehyde die nachfolgend aufgeführten Diarylimidazolderivate:

1a) 2-(4-Hydroxyphenyl)-4-(5)-(4-dimethylaminophenyl)-5-(4)-methyl-(1H)-imidazol-hydrochlorid, Fp. 242° C (Zers.), λ max 412 nm (ε = 21.860)

1b) 2-(3,5-Di-tert.butyl-4-hydroxyphenyl)-4-(5)-(4-dimethylaminophenyl)-5-(4)-methyl-(1H)-imidazol-hydrochlorid, Fp. 255° C (Zers.), λ max 650 nm (ε = 41.830)

1c) 2-(4-Hydroxyphenyl)-4-(5)-(3,5-dimethoxy-4-hydroxyphenyl)-5-(4)-methyl-(1H)-imidazol-hydrochlorid, Fp. 326° C (Zers.), λ max 533 nm (ε = 6.400)

1d) 2-(3,5-Di-tert.butyl-4-hydroxyphenyl)-4-(5)-(3,5-dimethoxy-4-hydroxyphenyl)-5-(4)-methyl-(1H)-imidazol-hydrochlorid, Fp. 296° C (Zers.), λ max. 673 nm (ε = 29.800)

1e) 2-(3,5-Dimethoxy-4-hydroxyphenyl)-4-(5)-(4-hydroxyphenyl)-5-(4)-methyl-(1H)-imidazol-hydrochlorid, Fp. 197-199° C (Zers.), λ max 533 nm (ε = 6.400)

1f) 2,4-(5)-Di-(3,5-Dimethoxy-4-hydroxyphenyl)-5-(4)-methyl-(1H)-imidazol-hydrochlorid, Fp. 158° C (Zers.), λ max. 590 nm (ε = 17.900)

1g) 2-(4-Dimethylaminophenyl)-4-(5)-(4-hydroxyphenyl)-5-(4)-methyl-(1H)-imidazol-hydrochlorid, Fp. 225-230° C (Zers.), λ max. 510 nm (ε = 10.500)

1h) 2-(4-Dimethylaminophenyl)-4-(5)-(3,5-dimethoxy-4-hydroxyphenyl)-5-(4)-methyl-(1H)-imidazol-hydrochlorid, Fp. 182-200° C (Zers.), λ max. 670 nm (ε= 10.800)

1i) 2-(6-N-Methyl-1,2,3,4-tetrahydrochinolino)-4-(5)-(3,5-dimethoxy-4-hydroxyphenyl)-5-(4)-methyl-(1H)-imidazol-hydrochlorid, Fp. 190° C (Zers.), λ max 700 nm (ε = 19.400)

1j) 2-(9-Juloli dino)-4-(5)-(3,5-dimethoxy-4-hydroxyphenyl)-5-(4)-methyl-(1H)-imidazol-hydrochlorid, Fp. 201° C (Zers.), λ max. 690 nm (ε = 25.399)

1k) 2-(4-Hydroxy-3-methoxyphenyl)-4-(5)-(4-hydroxyphenyl)-5-(4)-methyl-(1H)-imidazol-hydrochlorid, Fp. > 270° C (Zers.), λ max. 502 nm (ε = 3.730)

1l) 2-(3,5-Di-tert.-butyl-4-hydroxyphenyl)-4-(5)-(4-hydroxyphenyl)-5-(4)-methyl-(1H)-imidazol, Fp. 200-202° C (Zers.), λ max. 485 nm (ε = 6.700)

1m) 2-(3,5-Dimethoxy-4-hydroxyphenyl)-5-(4)-methyl-(1H-)-imidazolyl-4-(5)-(phenyl-4-amino-N-ethansulfonsäure), Fp. > 260° C (Zers.), λ max. 615 nm (ε = 27.500)

1n) 2-(3,5-Dimethoxy-4-hydroxyphenyl)-5-(4)-methyl-(1H)-imidazolyl-4-(5)-(phenyl-4-amino-N-ethanphosphonsäure), Fp. > 250° C (Zers.) λ max. 624 nm (ε = 25.600)

1o) 2-(3,5-Dimethoxy-4-hydroxyphenyl)-5-(4)-methyl-(1H)-imidazolyl-4-(5)-(phenyl-4-aminoessigsäure), Fp. 124° C (Zers.), λ max. 620 nm (ε = 18.800)

1p) 2-(3,5-Di-tert.butyl-4-hydroxyphenyl)-4-(5)-(4-amino-phenyl)-5-(4)-methyl-(1H) imidazol, Fp. 228-230° C, λ max. 576 nm (ε = 31.200)

1q) 2-(3,5-Di-tert.butyl-4-hydroxyphenyl)-5-(4)-methyl-(1H)-imidazolyl-4-(5)-(phenylaminoessigsäure) amorph λ max. 614 nm (ε = 39.900)

1r) 2-(3,5-Di-tert.butyl-4-hydroxyphenyl)-5-(4)-methyl-(1H)-imidazolyl-4-(5)-phenylamino-diessigsäure), Fp. 225° C (Zers.), λ max. 580 nm (ε= 31.700)

1s) 2-(1-Benzyl-1,2,3,4-tetrahydrochinolino)-4-(5)-(3,5-dimethoxy-4-hydroxyphenyl)-5-(4)-methyl-(1H)-imidazol, Fp. 190° C (Zers.), λ max. 705 nm (ε = 31.500)

**Beispiel 2**

2-(3,5-Dimethoxy-4-hydroxyphenyl)-4-(5)-(4-aminophenyl)-5-(4)-methyl-(1H)-imidazol-hydrochlorid

Man löst 11 g (0,05 Mol) 1-(4-Acetylaminophenyl)-1-oximino-2-propanon und 13,6 g (0,05 Mol) 4-Benzyloxy-3,5-dimethoxybenzaldehyd unter Zugabe von 10 g (0,13 Mol) Ammoniumacetat in 100 ml Eisessig und erhitzt die Reaktionsmischung zwei Stunden unter Rückfluß. Danach gibt man unter Rühren portionsweise 10 g Zinkstaub zu und kocht weitere zwei Stunden unter Argon. Nach Stehen über Nacht saugt man das

auskristallisierte Zinkacetat ab und versetzt das Filtrat unter Rühren und Kühlen langsam mit 0,5 l konz. Ammoniak. Die in Freiheit gesetzte Imidazolbase wird in Dichlormethan aufgenommen, über Natriumsulfat getrocknet und das Lösungsmittel am Rotationsverdampfer eingeengt. Man erhält 22,43 g Rohbase, bräunliche Kristalle. Dieses Produkt wird in 200 ml 6 N Chlorwasserstoffsäure gelöst und 45 min unter Rückfluß erhitzt. Beim Abkühlen kristallisieren 15,1 g (74,9 % dTh) der Titelverbindung farblose Kristalle. Fp. 250° C (Zers.)

λ max 580 nm (ε = 18.900)

2a) 2-[3,5-Dimethoxy-4-hydroxyphenyl]-4-(5)-(4-amino-3-methoxyphenyl-5-(4)-(1)-methyl-(1H)-imidazol-hydrochlorid

In gleicher Weise wie im vorstehenden Beispiel 2 beschrieben, erhält man aus 1-(4-Acetylamino-3-methoxyphenyl)-1-oximino-2-propanon und 3,5-Dimethoxy-4-hydroxy-benzaldehyd:2-[3,5-Dimethoxy-4-hydroxyphenyl]-4-(5)-(4-amino-3-methoxyphenyl)-5-(4)-methyl-(1H)-imidazol-hydrochlorid, farblose Kristalle, Fp. 218° C (Zers.)

λ max 402 nm (ε = 15.400)

**Beispiel 3**

2-(4-Aminophenyl)-4-(5)-(3,5-dimethoxy-4-hydroxyphenyl)-(1H)-imidazol-hydrochlorid

α) In einer Zweiphasenkondensation werden 4,0 g (0,02 Mol) 4-Nitrobenzamidin-hydrochlorid in 30 ml Wasser und 5,5 g (0,02 Mol) ω-Bromacetosyringon in 50 ml Chloroform bei 25°C unter heftigem Rühren mit 20 ml (0,04 Mol) einer 2-molaren wäßrigen Kaliumhydroxidlösung versetzt und anschließend ca. vier Stunden zum Sieden erhitzt. Nach Abtrennen der Chloroform-Phase wird die wäßrige Phase neutralisiert und mehrmals mit Chloroform extrahiert. Die vereinigten organischen Phasen werden eingeengt und der Rückstand säulenchromatographisch an Kieselgel mit Essigester als Elutionsmittel gereinigt.

1,2 g 2-(4-Nitrophenyl)-4-(5)-(3,5-dimethoxy-4-hydroxyphenyl)-(1H)-imidazol farbloses, amorphes Material, charakterisiert durch $^1$H-NMR Spektroskopie, EI-Massenspektrometrie und Elementaranalyse (Ausbeute 18 % d.Th.)

β) 1,0 g (0,003 Mol) der vorstehenden Nitroverbindung in 50 ml Ethanol werden in Gegenwart von 0,1 g 10 %igem Palladium-auf-Aktivkohle bei 25° C unter Normaldruch hydriert. Nach Abfiltrieren des Katalysators wird mit etherischem Chlorwasserstoff versetzt und eingeengt. Der Rückstand wird mit Ether verrieben und ergibt 0,7 g der Titelverbindung (Ausbeute 61 % d.Th.)

Fp. 285-290° C

λ max 560 nm (ε = 24.500)

**Beispiel 4**

2-(4-Dimethylaminophenyl)-4-(5)-(3,5-dimethoxy-4-hydroxyphenyl)-(1H) imidazol-hydrochlorid

α) Eine Suspension von 6,4 g (0,018 Mol)

4-Benzyloxy-3,5-dimethoxy-ω-bromacetophenon und 3,3 g (0,018 Mol) Natr ium-4-N,N-dimethylaminobenzoat in 150 ml absolutem Dimethylformamid werden unter Rühren 1,5 Stunden auf 130°C erwärmt. Abdampfen des Lösungsmittels und Anreiben des Rückstandes mit Dichlormethan und schließlich mit Ether ergibt

4,2 g 4-Benzyloxy-3,5-dimethoxy-ω-(4-N,N-dimethylaminbenzoyloxy)-acetophenon

farbloses pulver (Ausbeute 53 % d.Th.)

Fp. 114°C

β) 30 g (0,067 Mol) des nach vorstehendem Verfahren erhaltenen Esters werden in 150 ml Eisessig mit 51,4 g (0,67 Mol) Ammoniumacetat unter Rühren zwei Stunden auf 130°C erhitzt. Nach dem Erkalten wird auf ein Liter Eiswasser gegossen und mehrmals mit Essigester extrahiert. Die vereinigten Essigesterextrakte werden mit verdünnter wäßriger Natronlauge gewaschen, getrocknet und eingeengt. Der Rückstand wird säulenchromatographisch an Kieselgelgereinigt, zuerst mit Toluol/Essigester = 9/1, um weniger polare Komponenten abzutrennen, dann mir Toluol/Essigester/Methanol, 2/2/1. Einengen der entsprechenden Fraktion ergibt

5 g 2-(4-Dimethylaminophenyl)-4-(5)-(4-benzyloxy-3,5-dimethoxyphenyl-(1H)-imidazol

farblose Substanz (Ausbeute 18 % d.Th.)

Fp. 196°C

γ) 4,3 g (0,01 Mol) des erhaltenen Imidazolderivates werden in 100 ml Ethanol in Gegenwart von 0,3 g 10 %igem Palladium-auf-Aktivkohle bei 25°C unter Normaldruck hydriert. Nach Entfernen des Katalysators wird die Lösung mit 1 ml conc. Salzsäure versetzt, eingeengt und ergibt

3,2 g der Titelverbindung (Ausbeute 70 % d.Th.)

λ max 626 nm (ε = 4.770)

**Beispiel 5**

2,4-(5-)-Bis-(4-dimethylaminophenyl)-(1H)-imidazol-hydrochlorid

α) 4-Dimethylaminobenzaldehyd-Bisulfitaddukt

10 g (0,067 Mol) 4-Dimethylaminobenzaldehyd wurden in 50 ml Toluol gelöst und mit 100 ml gesättigter Bisulfitlösung sechs Stunden kräftig gerührt. Das entstehende kristalline Material wird abgesaugt und mit 50 ml Toluol gewaschen.

Ausbeute: 12,8 g (75 % d.Th.)

β) (4-Dimethylaminophenyl)-methylimino-(4-dimethylaminophenyl)-acetonitril

12,1 g (0,048 Mol) Bisulfidaddukt, 45 ml konz. Ammoniak und 2,6 g (0,053 Mol) Natriumcyanid werden 2 h bei 37°C gerührt und dann mit 3 x 75 ml Dichlormethan durchgeschüttelt, die organische Phase abgetrennt, dreimal mit je 20 ml Wasser nachgeschüttelt und eingeengt. Man erhält 7,5 g rötliches Öl, das nach Umkristallisieren aus 30 ml Isopropanol 3,8 g (26 % d.Th.) der Titelverbindung, farblose Kristalle, Fp. 144-146°C (Zers.) ergibt.

γ) 2,4-(5)-Bis-(4-dimethylaminophenyl)-(1H)-imidazol-hydrochlorid

3,5 g (0,011 Mol) Acetonitril-verbindung werden in 40 ml abs. Dioxan gelöst, zum Sieden erhitzt und unter Rühren zwei Stunden Chlorwasserstoffgas eingeleitet. Das dabei entstehende, amorphe Hydrochlorid wird abgetrennt und in 25 ml Wasser gelöst. Durch Zugabe von konz. Ammoniak und Durchschütteln mit 3 x 50 ml Dichlormethan erhält man 3,2 g amorphes Rohprodukt, das an einer Kieselgel-60-Säule mit Essigester-Chloroform 2/1 chromatographisch gereinigt wird. Die entsprechenden Fraktionen werden anschließend aus 20 ml Isopropanol umkristallisiert. Man isoliert 2,24 g (73,2 % d.Th.) der Titelverbindung; Fp. 233 bis 235°C.

λ max. 764 nm (ε = 9.800)

**Beispiel 6**

2-(3,5-Dimethoxy-4-hydroxyphenyl)-4-(5)-(4-dimethyl-aminophenyl-)-5-(4) (2-tetrahydrolfuryl)-(1H)-imidazol-hydrochlorid

α) 2-(3,5-Dimethoxy-4-hydroxyphenyl)-4-(5)-dimethylaminophenyl-5-(4)-(2-furyl)-(1H)-imidazol-hydrochlorid

72,9 g (0,3 Mol) 1,2-Dioxo-2-(4-dimethylaminophenyl)-1-furyl-(2)-ethan werden unter Argon mit 60,12 g (0,33 Mol) 3,5-Dimethoxy-4-hydroxybenzaldehyd (GC 98 %) und 231 g (3 Mol) Ammoniumacetat in 1,2 l Eisessig vier Stunden unter Rühren und Rückfluß erhitzt. Danach läßt man abkühlen und gießt, auf 3,6 l Eiswasser. Anschließend schüttelt man mit 4 x 500 ml Essigester aus und rührt die vereinigten Essigesterphasen 30 Minuten mit 50 g Zinkstaub. Nach Absaugen und Einengen der Imidazollösung im Vakuum löst man den Rückstand in 150 ml Methanol und tropft die Lösung unter Schutzgas in 1 l Wasser. Die gebildeten Kristalle werden abgesaugt und getrocknet. Man erhält 113,2 g leicht grüngefärbtes Imidazol, das an einer Kieselgelsäule weiter gereinigt wird.

Trennflüssigkeit: Chloroform/Methanol 12/1. Die entsprechenden Fraktionen enthalten 57,5 g Rohimidazol. Das nach Anrühren mit 300 ml Aceton 50 g (41,1 % d.Th.) farbloses Imidazol ergibt. Durch Elution der Säule mit Methanol und Aufarbeiten der Mutterlauge gewinnt man weitere 15,6 g schwach verunreinigtes Produkt. (Gesamtausbeute 54 % d.Th.). Bei Zugabe von 50 ml 5 N etherischer Chlorwasserstoffsäure zu einer Suspension der Imidazolbase in 100 ml Ethanol tritt zunächst Lösung, dann Kristallisation ein, die durch Einstellen in ein Eisbad vervollständigt wird. Man erhält 53,2 g fast farbloses Hydrochlorid der Titelverbindung; Fp. 172 °C (Zers.)

λ max 680 nm (ε = 12.342)

β) 10,9 g (0,021 Mol) der erhaltenen Verbindung werden in 250 ml p.a. Methanol gelöst und unter Zugabe von 1,5 g Palladiummohr sechs Stunden bei 36 bis 38°C unter Normaldruck hydriert. Man arbeitet wie üblich auf und reinigt das Rohprodukt durch Säulenchromatographie in Kieselgel 60 mit Chloroform/Methanol 5/1. Es resultieren 1,24 g (13,6 % d.Th.) beigefarbenes chromatographisch einheitliches Kristallisat der Titelverbindung.

Fp. 98°C/110°C (Zers.) (kein klarer Schmelzpunkt!) DC-Fertigplatte; Kieselgel; Laufmittel: Chloroform-Methanol 5/1,

$R_f = 0{,}36$

λ max 680 nm (ε = 16.700)

**Beispiel 7**

2-(3,5-Dimethoxy-4-hydroxyphenyl)-4-(5)-(4-dimethylaminophenyl)-5-(4) (4-hydroxybutyl)-(1H)-imidazol-hydrochlorid

16 g (0,023 Mol) der Titelverbindung Beisp. 6 werden in 400 ml Methanol unter Zugabe von 2 g Palladiummohr acht Stunden bei 36 bis 38 °C unter Normaldruck hydriert. Das nach Aufarbeitung anfallende Rohprodukt wird an Kieselgel 60 mit Chloroform-Methanol 5/1 gereinigt. Man erhält 4 g chromatographisch einheitliches

Material, das man in 50 ml Methanol löst, 30' mit 2 g Zinkstaub rührt und durch Zugabe von 20 ml 5 N etherischer Chlorwasserstoffsäure als Hydrochlorid isoliert. Es ergeben sich 3,52 g (21,8 % Ausbeute) der Titelverbindung, farblose Kristalle; Fp. 140/212°C (Zers.)

λ max 640 nm (ε = 25.000)

**Beispiel 8**

2-(3,5-Dimethoxy-4-hydroxyphenyl)-4-(5)-(4-dimethylaminophenyl)-5-(4) methyl-(1H)-imidazol-hydrochlorid

5 g (0,0126 Mol) der Titelverbindung Beisp. 2 werden in 140 ml Methanol suspendiert und nach Zugabe von 0,7 ml konz. Chlorwasserstoffsäure und 0,5 g Platinoxid sowie 3,3 ml 37 %iger Formalinlösung fünf Stunden bei 5 bis 10°C hydriert. Nach beendeter Wasserstoffaufnahme wird vom Katalysator abgesaugt, das Filtrat in Vakuum eingeengt und das Hydrierungsprodukt 5,95 g farblose Kristalle aus 150 ml Methanol umkristallisiert. Man erhält 4,2 g (70,1 % Ausb.) farblose Kristalle der Titelverbindung; Fp. 185/218 bis 219°C (Zers.)

λ max 680 nm (ε = 25.500)

8a) 2-(3,5-Dimethoxy-4-hydroxyphenyl)-4-(5)-(4-dimethylamino-3-methoxyphenyl)-5-(4)-methyl-(1H)-imidazol-hydrochlorid.

In analoger Weise erhält man, wie in Beisp. 8 beschrieben, aus dem Syntheseprodukt Beisp. 2 a die obengenannte Titelverbidung farblose Kristalle; Fp. 211°C (Zers.)

λ max 417 nm (ε = 28.300)

**Beispiel 9**

2-(3,5-Dimethoxy-4-hydroxyphenyl)-5-(4)-methyl-(1H)-imidazolyl-4-(5)-(phenyl-4-amino-N-mono-, bzw. bis-ethan-2-sulfonsäure)

3,3 g (0,01 Mol) 2-(3,5-Dimethoxy-4-hydroxyphenyl)-4-(5)-(4-aminophenyl)-5-(4)-methyl-(1H)-imidazol (Base aus Beisp. 2) werden in 50 ml DMF gelöst, 2,11 g (0,01 Mol) 2-Bromethan-sulfonsäure, Natriumsalz zugegeben und 7 Stunden unter Argon am Rückfluß erhitzt. Nach Abdampfen des Lösungsmittels im Vakuum wird der ölige Rückstand an einer Kieselgel-60-Säule (Füllhöhe 110 cm, Ø 5 cm) chromatografisch gereinigt. Laufmittel: Isopropanol/n-Butylether/Wasser im Verhältnis 5:3:2. Durch Einengen der entsprechenden Fraktionen erhält man Rohprodukte, die nach Aufkochen mit Wasser jeweils 1,3 g der Mono- und 0,6 g der Bis-Titelverbindung ergeben.

DC-Fertigplatte Kieselgel 60-F-254

$R_f$-Wert der Monoverbindung: 0,44

UV: λ max 620 (ε = 29.100)

$R_f$-Wert der Bisverbindung: 0,28

UV: λ max 610 (ε = 28.400)

**Beispiel 10**

Übersicht über die optischen Eigenschaften der Substanzen der allgemeinen Formel 1

Zur Ermittlung des molaren Extinktionskoeffizienten wird wie folgt vorgegangen: 2 x $10^{-2}$ mol Indikator der allgemeinen Formel I werden in 100 ml 0,1 m Salzsäure gelöst. Löst sich eine Substanz nicht quantitativ auf, wird in einem Gemisch Salzsäure, Methanol 9:1 gelöst. Von dieser Lösung werden 0,1 ml mit 10 ml 0,1 m Phosphatpuffer pH 6,0 verdünnt. Von der so erhaltenen Indikatorlösung werden 10 µl in ein Gemisch, bestehend aus 10 µl verdünntes $H_2O_2$ (100 µl 30 %iges $H_2O_2$ werden auf 100 ml mit Wasser verdünnt), 10 µl Peroxidaselösung (600 U Peroxidase werden in 1 ml Wasser gelöst) und 10 ml 0,1 m Phosphatpuffer pH 6,0 pipettiert. Der Indikator wird oxidiert, die Lösung verfärbt sich. Von der gefärbten Lösung wird nach 60 sec ein Spektrum geschrieben und aus den Extinktionswerten die molare Extinktionskoeffizienten berechnet (fällt der gebildete Farbstoff aus, so wird ein gemisch aus Puffer, Aceton oder Methanol 9:1 verwendet).

Nach dem gleichen Verfahren lassen sich auch aus Proben die $H_2O_2$ Konzentrationen bzw. die Konzentrationen von Substraten ermitteln aus denen durch eine vorgeschaltete enzymatische Reaktion als ein Reaktionsprodukt $H_2O_2$ entsteht.

Beispiele für die Einsetzbarkeit der erfindungsgemäßen Redoxindikatoren:

**Beispiel 11**

Testsystem zum Nachweis von Harnsäure in wässrigen Lösungen

Auf eine mit Gelatine vorbeschichtete Polyesterfolie wird mit einer Naßfilmdicke von 300 µm eine Gelatinematrix der unten aufgeführten Zusammensetzung gegossen und anschließend getrocknet. In 47,5 ml Tris-Phosphat-Puffer 0,5 m, pH 7,2 werden 8,4 g Gelatine, 0,25 g Polyoxyethylen-sorbitanmonolaurat (Tween 20[R]), 0,5 KU (KU = Kilounits) Uricase, 0,5 KU Peroxidase und 100 mg Indikator-Substanz aus Beispiel 7, d.h. 2-(3,5-Dimethoxy-4-hydroxyphenyl)-4-(5)-(4-dimethylaminophenyl)-5-(4)-(4-hydroxybutyl)-(1H)-imidazol-hydrochlorid, eingearbeitet. Der so hergestellte Reagenzfilm wird zu einem Testsystem entsprechend Abb. 1 weiterverarbeitet.

Auf die Dosierzone werden 35 µl Harnsäurelösung aufgebracht. Durch Andrücken der Reagenzzone und des Gewebes auf die Transportzone wird die Reaktion gestartet. Nach zwei Minuten wird in einem Remissionsphotometer gemessen (das Gewebe hat die Funktion, die Unebenheiten des Glasfaservlieses zu egalisieren).

Die mit dem beschriebenen System erhaltenen Eichkurven werden in der folgenden Tabelle wiedergegeben:

| Harnsäurekonzentration | % Remission |
|---|---|
| 3 mg/dl | 55,1 |
| 5 mg/dl | 49,3 |
| 7 mg/dl | 43,8 |
| 9 mg/dl | 39,5 |
| 11 mg/dl | 34,2 |
| 14 mg/dl | 31,3 |

**Beispiel 12**

Testsystem zum Nachweis von Creatinin im Serum

Ein saugfähiger Träger (Schablonenpapier der Firma Schöller und Hösch, Flächengewicht 12 $g/m^2$, Saugfähigkeit 50 $ml/m^2$) wird mit einer Lösung von 200 KU Peroxidase und 1,2 g Kollagenhydrolysat gelöst in 100 ml Phosphatpuffer 0,1 m, pH 8,0 imprägniert und getrocknet. In einem zweiten Imprägnierungsvorgang wird das vorimprägnierte Papier mit einer Lösung bestehend aus 2 mmol Indikator-Substanz aus Beispiel 7 in 100 ml Methanol nachimprägniert und getrocknet. Man erhält das Reagenzpapier (8).

Zur Herstellung des Reagenzpapiers (9) wird der oben genannte saugfähige Träger mit einer Lösung von 5 KU Sarkosinoxidase, 30 KU Creatininamidohydrolase und 40 KU Creatininamidinohydrolase, 0,5 g Alkylphenol-polyethylenglycolether (Triton X 100[R]) in 100 ml 0,1 m Phosphatpuffer pH 8,0 imprägniert und getrocknet.

Beide Papiere werden entsprechend der Abbildung 2 in ein Testsystem eingearbeitet.

Zum Nachweis von Creatinin im Serum pipettiert man 30 µl Serum auf die Dosierzone. Durch das Andrücken des Enzym- und Indikatorpapiers auf die Transportzone wird die Reaktion gestartet. Die entsprechende Farbe wird nach einer Minute remissionsphotometrisch vermessen. Die Auswertung erfolgt über eine Eichkurve.

In der folgenden Tabelle werden die Werte für eine Eichkurve für Creatinin im Serum wiedergegeben:

| Creatininkonzentration | % Remission |
|---|---|
| 0,1 mg/dl | 68,0 |
| 0,5 mg/dl | 57,2 |
| 1,5 mg/dl | 45,1 |
| 5,0 mg/dl | 32,7 |
| 10,0 mg/dl | 26,4 |

**Beispiel 13**

Testsystem zum Nachweis von Harnsäure in Blut

Aus den unten aufgeführten Bestandteilen wird eine Beschichtungsmasse hergestellt und mit einer Naßfilmdicke von 200 µm auf eine transparente Folie aufgerakelt und getrocknet. 18 g einer Kunststoffdispersion eines Mischpolymeren aus Vinylacetat und Vinylpropionat, 1,38 g Alginat, 69 g eines Tris-Citrat-Puffers pH 7,5, 0,45 m, 0,47 g Indikator 2-(3,5-Dimethoxy-4-hydroxyphenyl)-4-(5)-(4-dimethylaminophenyl)-5-(4)-methyl-(1H)-imidazol-hydrochlorid (Substanz aus Beispiel 1) 0,025 g 1-(3-Chlorphenyl)-semicarbazid, 0,025 g $MgK_2EDTA$ . $2H_2O$, 0,5 g Alkylphenol-polyethylenglycolether (Triton X 100[R]), 0,6 g Hexanol, 200 KU Peroxidase, 2 KU Uricase.

Auf die so hergestellte Schicht wird eine zweite Schicht als optisch weißer Hintergrund der unten aufgeführten Zusammensetzung mit einer Schichtdicke von 200 µm gerakelt und getrocknet. 52 ml 0,1 m Tris-Citrat-Puffer pH 7,0, 5,5 g Titandioxid, 2,7 g Diatomeenerde, 0,4 g Alginat, 1,4 g einer Kunststoffdispersion eines Mischpolymerenzusatzes aus Vinylacetat und Vinylpropionat 0,2 g Alkylphenolpolyethylenglycolether (Triton X 100R)

Der so hergestellte Testfilm wird zu Testen gemäß Abbildung 3 verarbeitet.

Zum Nachweis von Harnsäure aus Blut werden 30 µl Blut auf die Dosierzone aufgetragen, die Reagenzklappe wird nach einer Minute angedrückt und nach weiteren zwei Minuten wird mit einem Remissionsphotometer die gebildete Farbe vermessen und aus einer vorher ermittelten Eichkurve die Harnsäurewerte ermittelt.

In der folgenden Tabelle werden die Werte für die Eichkurve wiedergegeben:

| Harnsäurekonzentration | % Remission |
|---|---|
| 4,6 | 53,0 |
| 7,9 | 40,3 |
| 9,8 | 35,0 |
| 13,5 | 28,3 |
| 20,2 | 19,8 |

**Beispiel 14**

Testsystem zum Nachweis von GOT (Glutamat-Oxalacetat-Transaminase) Blut

Je ein saugfähiger Träger (Schablonenpapier der Firma Schöller und Hösch, Flächengewicht 12 $g/m^2$, Saugfähigkeit 50 $ml/m^2$) wird mit den unten aufgeführten Lösungen 1 und 2 imprägniert und getrocknet.

Lösung 1: In einem Liter eines 0,2 m Puffers aus Kalilauge und 2-(N-Morpholino)-äthansulfonsäure von pH 6,7 werden gelöst: 0,03 mol α-Ketoglutarat, 0,8 mol Alaninsulfinsäure, 0,01 mol Magnesiumchlorid, 0,0001 mol Ascorbinsäure, 0,009 mol Substanz aus Beispiel 1 und 5 g Octylpyranosid. Man erhält das Reagenzpapier (8)

Lösung 2: In einem Liter des obengenannten Puffers werden gelöst: 0,003 mol Thiaminpyrophosphat, 500 KU Pyruvatoxidase, 500 KU Peroxidase, 100 KU Ascorbatoxidase. Man erhält das Reagenzpapier (9).

Diese Reagenzpapiere werden zu einem Testsystem gemäß Abbildung 2 verarbeitet.

Zur Bestimmung der Enzymaktivität werden 30 µl Blut auf die Dosierzone pipettiert, nach einer Minute die Deckfolie und die Reaktionspapiere angedrückt und die Farbentwicklung nach der Zeit mit einem Remissionsphotometer verfolgt. Die Auswertung erfolgt über eine Zweipunktmessung aus einer Bezugskurve. Die Bezugskurve wird erstellt, in dem man Verdünnungsreihen mit Enzymaktivitäten von 10 - 1000 U/l herstellt und im Remissionsphotometer die Remissionswerte über Fixtime-messungen erarbeitet.

**Beispiel 15**

Verfahren zum Nachweis von niedrigen Glucosekonzentrationen im Blut zur Diagnose einer Hypoglycämie

Eine Rohfilmmasse wird wie folgt hergestellt.

10 g einer 1,7 %igen Alginatanquellung in einem 0,5 m Phosphatpuffer pH 5,0, 15 g wässrige Kunststoffdispersion eines Copolymeren aus Vinylacetat und Vinylpropionat, 5 g einer 15 %igen wässrigen Lösung von 4-Dodecylbenzolsulfonat, 25 KU Glucoseoxidase, 200 KU Peroxidase, 270 mg Substanz aus Beispiel 1, 10 g Diatomeenerde, 0,4 ml Hexanol werden zu einem homogenen Brei verrührt und mit einer Naßfilmdicke von 150 µ auf ein multifiles Gewebe (2 F/964 der Firma Schweizer-Seidengaze Fabrik) aufgerakelt und anschließend getrocknet.

Dieser Film wird zu einem Testsystem gemäß Abbildung 4 verarbeitet. Zur Bestimmung der Glucose pipettiert man 30 µl Blut auf die Dosierzone, drückt die Deckfolie und den Reagenzfilm auf die Transportzone und vermißt die entstandene Reaktionsfarbe mit einem Remissionsphotometer. Die Glucosekonzentrationen werden anhand einer Eichkurve ermittelt, die das unten angegebene Aussehen hat:

| mg Glucose/dl | % Remission |
|---|---|
| 20 | 28,7 |
| 40 | 17,6 |
| 60 | 12,4 |
| 80 | 9,6 |

**Bezugszeichenliste**:

1. Reagenzzonen-Trägerfolie (transparent)
2. Reagenzzone
3. Gewebe
4. Vlies aus Glasfasern
5. Abtrennzone aus Glasfasern
6. Fixiernetz
7. Deckfolie (transparent)
8. Reagenzpapier a)
9. Reagenzpapier b)
10. Optisch weißer Hintergrund
11. Multifiles Gewebe
12. Trägerfolie
13. Klebestelle

**Patentansprüche** für die Vertragsstaaten AT, BE, CH, FR, IT, LI, LU, NL, SE.

1. Imidazolderivate der allgemeinen Formel I

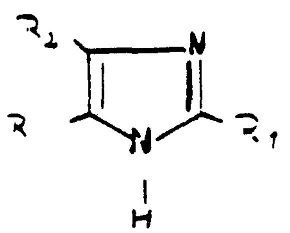

(I)

in der
R Wasserstoff, Tetrahydrofuranyl, Cycloalkyl oder Alkyl, das durch Hydroxy, Alkoxy, einen Schwefelsäure-, Phosphonsäure- oder Carbonsäurerest sowie eine Phenylgruppe substituiert sein kann,
und
$R_1$ und $R_2$, die gleich oder verschieden sein können,
einen Julolidin-Rest, einen Tetrahydrochinolin-Rest, der gegebenenfalls am Stickstoffatom eine Alkylgruppe tragen kann, die wiederum durch einen Schwefelsäure-, Phosphonsäure- oder Carbonsäure-Rest substituiert sein kann,
oder einen Rest

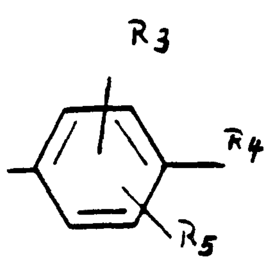

bedeuten,
in dem $R_4$ eine Hydroxy-, Amino-, eine mono- oder dialkylierte Aminogruppe, wobei die alkylreste einen Schwefelsäure-, Phosphonsäure- oder Carbonsäure-Rest tragen können,
und

$R_3$ und $R_5$, die gleich oder verschieden sein können Wasserstoff, Alkyl oder Alkoxy das gegebenenfalls mit einer Carboxylgruppe substituiert sein kann, darstellen,

mit der Maßgabe, daß $R_1$ und $R_2$ nicht gleichzeitig Julolidin- oder Tetrahydrochinolin sein dürfen und mindestens ein $R_4$ der Substituenten $R_1$ und $R_2$ eine Hydroxygruppe sein muß,

sowie deren Salze.

2. Verfahren zur Herstellung von Verbindungen gemäß Anspruch 1
dadurch gekennzeichnet, daß man
a) ein α-Diketon der Formel II

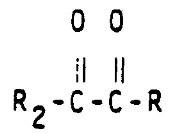

(II)

in der
$R_2$ und R die gleiche Bedeutung wie in Formel I besitzen mit einem Aldehyd der Formel III
$O=CH-R_1$ (III)
in dem
$R_1$ die gleiche Bedeutung wie in der Formel I besitzt, mit Ammoniak in saurer Lösung kondensiert
oder
b) ein α-Ketoxim der Formel IIa

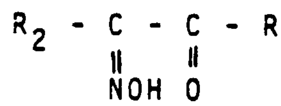

(IIa)

in der
$R_2$ und R die gleiche Bedeutung wie in Formel I besitzen, mit einem Aldehyd der Formel III und Ammoniak zu einer Verbindung der Formel Ia

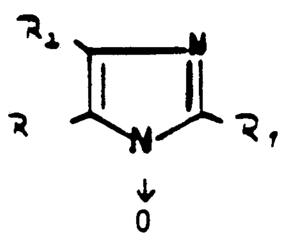

(Ia)

umsetzt und diese Verbindung in an sich bekannter Weise reduziert, oder
c) ein Acylbromid der Formel IV
$R_2$-CO-CHR Br (IV)
in der
$R_2$ und R die gleiche Bedeutung wie in Formel I besitzen mit einem Amidin der Formel V

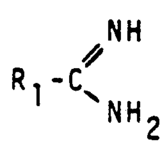

(V)

in der
$R_1$ die gleiche Bedeutung wie in der Formel I besitzt, in alkalischem Medium umsetzt
und anschließend, falls erforderlich, die erhaltenen Verbindungen in Verbindungen der Formel I überführt sowie entsprechende Imidazolbasen in Salze oder die Salze in die freien Basen umwandelt.

3. Verwendung von Imidazolderivaten gemäß Anspruch 1 als Redoxindikatoren.

4. Redoxindikator gemäß Anspruch 3 zum Nachweis von Wasserstoffperoxid oder peroxidatisch wirksamen Substanzen.

5. Reagenz zum Nachweis von Wasserstoffperoxid oder peroxidatisch wirksamen Substanzen, bestehend aus einer Verbindung der Formel I und üblichen Wirk- und Hilfsstoffen.

6. Reagenz gemäß Anspruch 5, dadurch gekennzeichnet, daß es in Form von Tabletten, Lyophylisaten, imprägnierten Reagenzträgern oder Reagenzfilmen Verwendung findet.

**Patentansprüche** für die Vertragsstaaten DE und GB.

1. Imidazolderivate der allgemeinen Formel I

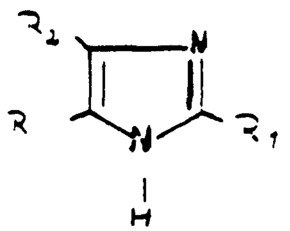

(I)

in der

R Wasserstoff, Tetrahydrofuranyl, Cycloalkyl oder Alkyl, das durch Hydroxy, Alkoxy, einen Schwefelsäure-, Phosphonsäure- oder Carbonsäurerest sowie eine Phenylgruppe substituiert sein kann, und

$R_1$ und $R_2$, die gleich oder verschieden sein können,

einen Julolidin-Rest, einen Tetrahydrochinolin-Rest, der gegebenenfalls am Stickstoffatom eine Alkylgruppe tragen kann, die wiederum durch einen Schwefelsäure-, Phosphonsäure- oder Carbonsäure-Rest substituiert sein kann,

oder einen Rest

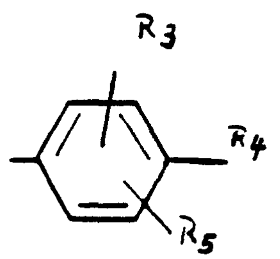

bedeuten, in dem

$R_4$ eine Hydroxy-, Amino-, eine mono- oder dialkylierte Aminogruppe, wobei die Alkylreste einen Schwefelsäure-, Phosphonsäure- oder Carbonsäure-Rest tragen können, und

$R_3$ und $R_5$, die gleich oder verschieden sein können, Wasserstoff, Alkyl oder Alkoxy, das gegebenenfalls mit einer Carboxylgruppe substituiert sein kann, darstellen,

sowie deren Salze

mit der Maßgabe, daß

$R_1$ und $R_2$ nicht gleichzeitig Julolidin- oder Tetrahydrochinolin sein dürfen, mindestens ein $R_4$ der Substituenten $R_1$ und $R_2$ eine Hydroxygruppe sein muß und solche Verbindungen der allgemeinen Formel I

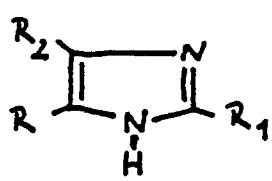

(I)

ausgenommen sind, in denen

$R_1$ und $R_2$ einen Rest

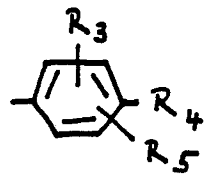

in dem $R_3$ Wasserstoff oder Alkoxy,
$R_4$ Hydroxy, Monoalkylamino oder Dialkylamino und
$R_5$ Wasserstoff oder Alkoxy
darstellen und
R einen Cycloalkyl- oder mehr als 2 C-Atome enthaltenden Alkylrest, die gegebenenfalls durch Hydroxy, Alkoxy, Phenyl oder durch eine Carboxylgruppe substituiert sind,
bedeuten.

2. Verfahren zur Herstellung von Verbindungen gemäß Anspruch 1
dadurch gekennzeichnet, daß man
a) ein α-Diketon der Formel II

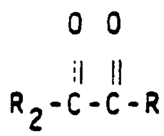 (II)

in der
$R_2$ und R die gleiche Bedeutung wie in Formel besitzen mit einem Aldehyd der Formel III
$O=CH-R_1$ (III)
in dem
$R_1$ die gleiche Bedeutung wie in der Formel I besitzt, mit Ammoniak in säurer Lösung kondensiert
oder
b) ein α-Ketoxim der Formel IIa

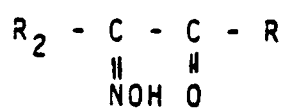 (IIa)

in der
$R_2$ und R die gleiche Bedeutung wie in Formel I besitzen, mit einem Aldehyd der Formel III und Ammoniak zu einer Verbindung der Formel Ia

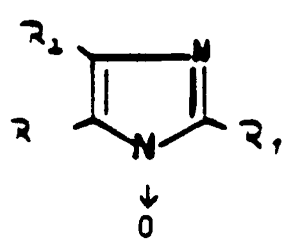 (Ia)

umsetzt und diese Verbindung in an sich bekannter Weise reduziert,
oder
c) ein Acylbromid der Formel IV
$R_2$-CO-CHR Br (IV)
in der
$R_2$ und R die gleiche Bedeutung wie in Formel I besitzen mit einem Amidin der Formel V

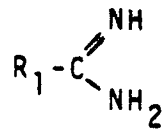

(V)

in der
$R_1$ die gleiche Bedeutung wie in der Formel I besitzt, in alkalischem Medium umsetzt
und anschließend, falls erforderlich, die erhaltenen Verbindungen in Verbindungen der Formel I überführt sowie entsprechende Imidazolbasen in Salze oder die Salze in die freien Basen umwandelt.

3. Verwendung von Imidazolderivaten gemäß Anspruch 1 als Redoxindikatoren.

4. Redoxindikator gemäß Anspruch 3 zum Nachweis von Wasserstoffperoxid oder peroxidatisch wirksamen Substanzen.

5. Reagenz zum Nachweis von Wasserstoffperoxid oder peroxidatisch wirksamen Substanzen, bestehend aus einer Verbindung der Formel I und üblichen Wirk- und Hilfsstoffen.

6. Reagenz gemäß Anspruch 5, dadurch gekennzeichnet, daß es in Form von Tabletten, Lyophylisaten, imprägnierten Reagenzträgern oder Reagenzfilmen Verwendung findet.

**Claims** for the contracting states: AT, BE, CH, FR, IT, LI, LU, NL, SE.

1. Imidazole derivativea of the general formula I

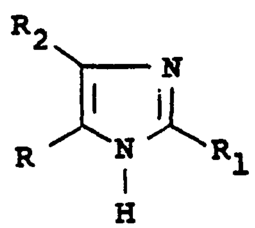

(I)

in which R signifies hydrogen, tetrahydrofuranyl, cycloalkyl or alkyl, which can be substituted by hydroxyl, alkoxy, a sulphuric acid, phosphonic acid or carboxylic acid residue, as well as by a phenyl group, and $R_1$ and $R_2$, which can be the same or different, signify a julolidine radical, a tetrahydroquinoline radical, which can optionally carry on the nitrogen atom an alkyl group which, in turn, can be substituted by a sulphuric acid, phosphonic acid or carboxylic acid residue, or a radical

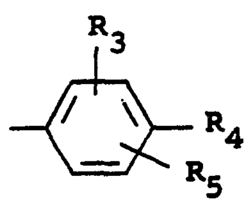

in which $R_4$ represents a hydroxyl, amino, a mono- or dialkylated amino group, whereby the alkyl radicals can carry a sulphuric acid, phosphonic acid or carboxylic acid residue, and $R_3$ and $R_5$, which can be the same or different, represent hydrogen, alkyl or alkoxy, which can optionally be substituted by a carboxyl group, with the proviso that $R_1$ and $R_2$ cannot simultaneously be julolidine or tetrahydroquinoline and at least one $R_4$ of the substituents $R_1$ and $R_2$ must be a hydroxyl group; as well as their salts.

2. Process for the preparation of compounds according to claim 1, characterised in that one
a) condenses an α-diketone of the formula II

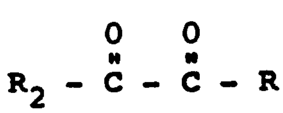

(II)

in which $R_2$ and R possess the same meaning as in formula I, with an aldehyde of the formula III
$O=CR-R_1$ (III)
in which $R_1$ possesses the same meaning as in formula I, with ammonia in acid solution or
b) reacts an α-ketoxime of the formula IIa

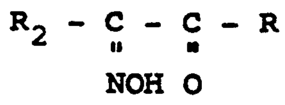

(IIa)

in which $R_2$ and R possess the same meaning as in formula I, with an aldehyde of the formula III and ammonia to a compound of the formula Ia

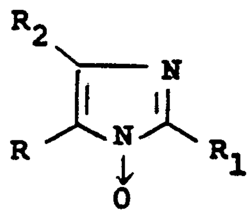

(Ia)

and reduces this compound in per se known manner, or
c) reacts an acyl bromide of the formula IV
$R_2$-CO-CHR-Br (IV)
in which $R_2$ and R possess the same meaning as in formula I, with an amidine of the formula V

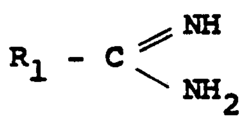

(V)

in which $R_1$ possesses the same meaning as in formula I, in alkaline medium, and subsequently, if necessary, converts the compounds obtained into compounds of the formula I, as well as converts corresponding imidazole bases into salts or converts the salts into the free bases.

3. Use of imidazole derivatives according to claim 1 as redox indicators.

4. Redox indicators according to claim 3 for the detection of hydrogen peroxide or of peroxidate-active substances.

5. Reagent for the detection of hydrogen peroxide or of peroxidate-active substances, consisting of a compound of the formula I and conventional active and adjuvant substances.

6. Reagent according to claim 5, characterised in that it finds use in the form of tablets, lyophilisates, impregnated reagent carriers or reagent films.

**Claims** for the contracting states: DE and GB

1. Imidazole derivatives of the general formula I

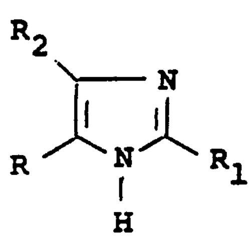

(I)

in which R signifies hydrogen, tetrahydrofuranyl, cycloalkyl or alkyl, which can be substituted by hydroxyl, alkoxy, a sulphuric acid, phosphonic acid or carboxylic acid residue, as well as by a phenyl group, and $R_1$ and $R_2$, which can be the same or different, signify a julolidine radical, a tetrahydroquinoline radical, which can optionally carry on the nitrogen atom an alkyl radical which, in turn, can be substituted by a sulphuric acid, phosphonic acid or carboxylic acid residue, or a radical

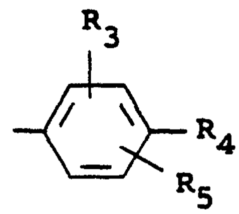

in which $R_4$ is a hydroxyl, amino, a mono- or dialkylated amino group, whereby the alkyl radicals can carry a sulphuric acid, phosphonic acid or carboxylic acid residue, and $R_3$ and $R_5$, which can be the same or different, represent hydrogen, alkyl or alkoxy, which are optionally substituted by a carboxyl group, as well as their salts, with the proviso that $R_1$ and $R_2$ cannot simultaneously be julolidine or tetrahydroquinoline, at least one $R_4$ of the substituents $R_1$ and $R_2$ must be a hydroxyl group and those compounds of the general formula I

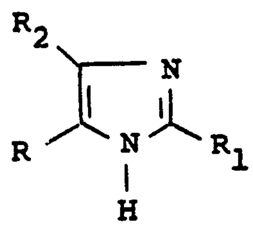

(I)

are excluded in which $R_1$ and $R_2$ signify the radical

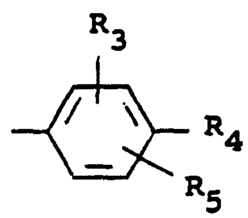

in which $R_3$ represents hydrogen or alkoxy, $R_4$ hydroxyl, monoalkylamino or dialkylamino and $R_5$ hydrogen or alkoxy and R signifies a cycloalkyl radical or an alkyl radical containing more than 2 C-atoms which are optionally substituted by hydroxyl, alkoxy, phenyl or by a carboxyl group.

2. Process for the preparation of compounds according to claim 1, characterised in that one

a) condenses an α-diketone of the formula II

$$R_2 - \overset{O}{\overset{\|}{C}} - \overset{O}{\overset{\|}{C}} - R \qquad \text{(II)}$$

in which $R_2$ and R possess the same meaning as in formula I, with an aldehyde of the formula III

$O{=}CH\text{-}R_1$ (III)

in which $R_1$ possesses the same meaning as in formula I, with ammonia in acid solution or

b) reacts an α-ketoxime of the formula IIa

$$R_2 - \underset{NOH}{\underset{\|}{C}} - \underset{O}{\underset{\|}{C}} - R \qquad \text{(IIa)}$$

in which $R_2$ and R possess the same meaning as in formula I, with an aldehyde of the formula III and ammonia to a compound of the formula Ia

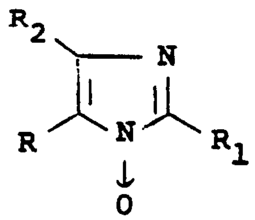

(Ia)

and reduces this compound in per se known manner or
c) reacts an acyl bromide of the formula IV
$R_2$-CO-CHR Br (IV)
in which $R_2$ and R possess the same meaning as in formula I, with an amidine of the formula V

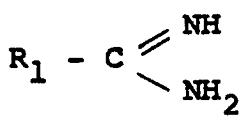

(V)

in which $R_1$ possesses the same meaning as in formula I, in alkaline medium, and subsequently, if necessary, converts the compounds obtained into compounds of the formula I, as well as converts corresponding imidazole bases into salts or converts the salts into the free bases.

3. Use of imidazole derivatives according to claim 1 as redox indicators.

4. Redox indicators according to claim 3 for the detection of hydrogen peroxide or of peroxidate-active substances.

5. Reagent for the detection of hydrogen peroxide or of peroxidate-active substances, consisting of a compound of the formula I and conventional active and adjuvant substances.

6. Reagent according to claim 5, characterised in that it finds use in the form of tablets, lyophilisates, impregnated reagent carriers or reagent films.

**Revendications** pour les Etats contractants: AT, BE, CH, FR, IT, LI, LU, NL, SE.

1. Dérivés d'imidazole de la formule générale I:

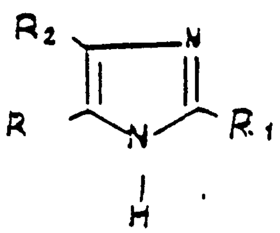

(I)

dans laquelle:

R représente l'hydrogène, un groupe tétrahydrofurannyle, cycloalkyle ou alkyle qui peut être substitué par un radical hydroxyle, alcoxyle, acide sulfurique, acide phosphonique ou acide carboxylique, ainsi que par un groupe phényle; et,

$R_1$ et $R_2$, qui peuvent être identiques ou différents, représentent un radical julolidine, un radical tétrahydroquinoléine qui peut évantuellement porter sur l'atome d'azote, un groupe alkyle, qui peut à nouveau être substitué par un radical acide sulfurique, acide phosphonique ou acide carboxylique,

ou un radical:

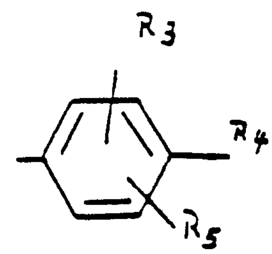

dans lequel:

$R_4$ représente un groupe hydroxyle, amine, un groupe amine mono- ou dialkylé, les radicaux alkyle pouvant porter un radical acide sulfurique, acide phosphonique ou acide carboxylique; et,

$R_3$ et $R_5$, qui peuvent être identiques ou différents, représentent l'hydrogène, un groupe alkyle ou alcoxyle qui peut éventuellement être substitué par un groupe carboxyle,

étant entendu que $R_1$ et $R_2$ ne peuvent pas être simultanément julolidine ou tétrahydroquinoléine, et qu'au moins un $R_4$ des substituants $R_1$ et $R_2$ doit être un groupe hydroxyle, ainsi que leurs sels.

2. Procédé pour la préparation de composés salon la revendication 1, caractérisé par le fait:

a) que l'on condense une α-dicétone de la formule II:

$$R_2-\overset{\overset{O}{\|}}{C}-\overset{\overset{O}{\|}}{C}-R \qquad (II),$$

dans laquelle:

$R_2$ at R ont la même signification que dans la formule I, avec un aldéhyde de la formula III:

$O = CH-R_1$ (III),

dans laquelle:

$R_1$ a la même signification que dans la formule I, et avec l'ammoniac en solution acide;

ou:

b) que l'on fait réagir une α-cétoxime de la formule IIa:

$$R_2-\underset{\underset{NOH}{\|}}{C}-\underset{\underset{O}{\|}}{C}-R \qquad (IIa)$$

dans laquelle:

$R_2$ et R ont la même signification que dans la formule I, avec un aldéhyde de la formule III et l'ammoniac, pour obtenir un composé de la formule Ia:

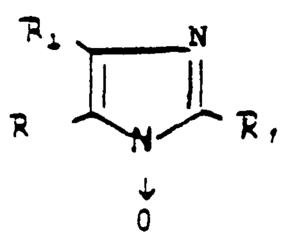

(Ia)

et que l'on reduit ce composé de manière en elle-même connue;

ou,

c) que l'on fait réagir en milieu alcalin un bromure d'acyle de la formula IV:

$R_2$-CO-CHR Br (IV),

dans laquelle:

$R_2$ et R ont la même signification que dans la formule I, sur une amidine de la formule V:

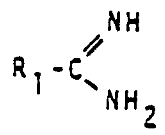

(V)

dans laquelle:

$R_1$ a la même signification que dans la formule I,

et qu'ensuite, si nécessaire, on convertit les composés obtenus en composés de la formule I, et que l'on convertit des bases imidazole correspondantes en sels, ou les sels an bases libres.

3. Utilisation de dérivés d'imidazole selon la revendication 1 comme indicateurs redox.

4. Indicateur redox selon la revendication 3, pour le décèlement de peroxyde d'hydrogène ou de substances ayant une action de peroxydase.

5. Réactif pour le décèlement de peroxyde d'hydrogène ou de substances ayant une action de peroxydase, formé d'un composé de la formule 1 et de substancas actives et adjuvants usuels.

6. Réactif selon la revandication 5, caractérisé par le fait qu'on l'utilise sous forme de comprimés, lyophilisats, supports de réactif imprégnés ou pellicules de réactif.

**Revendications** pour les Etats contractants DE, GB.

1. Dérivés d'imidazole de la formule générale I:

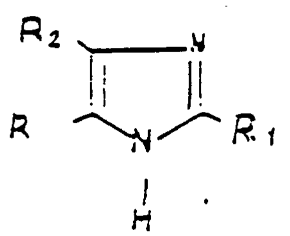

(I)

dans laquelle:

R représente l'hydrogène, un groupe tétrahydrofurannyle, cycloalkyle ou alkyle qui peut être substitué par un radical hydroxyle, alcoxyle, acide sulfurique, acide phosphonique ou acide carboxylique, ainsi que par un groupe phényle; et,

$R_1$ et $R_2$, qui peuvent être indentiques ou différents, représentant un radical julolidine, un radical tétrahydroquinoléine qui peut éventuellement porter sur l'atome d'azote, un groupe alkyle, qui peut à nouveau être substitué par un radical acide sulfurique, acide phosphonique ou acide carboxylique,

ou un radical:

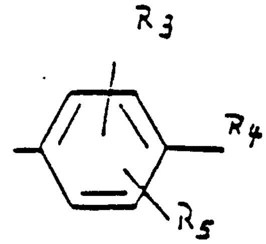

dans lequel:

$R_4$ représente un groupe hydroxyle, amine, un groupe amine mono- ou dialkylé, les radicaux alkyle pouvant porter un radical acide sulfurique, acide phosphonique ou acide carboxylique; et,

$R_3$ et $R_5$, qui peuvent être identiques ou differents, représentent l'hydrogène, un groupe alkyle ou alcoxyle qui peut éventuellement être substitué par un groupe carbonyle,

ainsi que leurs sels,

étant entendu que $R_1$ et $R_2$ ne peuvent pas être simultanément julolidine ou tétrahydroquinoléine,

qu'au moins un $R_4$ des substituants $R_1$ et $R_2$ doit être un groupe hydroxyle, et que sont exceptés les composés de la formule générale I:

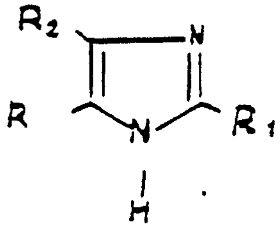

(I)

dans lesquels:
$R_1$ et $R_2$ représentent un radical:

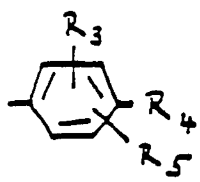

dans lequel:
$R_3$ représente l'hydrogène ou un groupe alcoxyle;
$R_4$ un groupe hydroxyle, monoalkylamine ou dialkylamine; et,
$R_5$ l'hydrogène ou un groupe alcoxyle, et,
R un radical cycloalkyle ou un radical alkyle contenant plus de 2 atomes de carbone, qui sont éventuellement substitués par des groupes hydroxyle, alcoxyle, phényle, ou par un groupe carboxyle.

2. Procédé pour la préparation de composés selon la revendication 1, caractérisé par le fait:
a) que l'on condense une α-dicétone de la formule II:

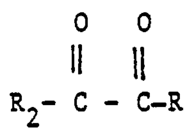

(II),

dans laquelle:
$R_2$ et R ont la même signification que dans la formule I, avec un aldéhyde de la formule III:
$O = Ch\text{-}R_1$ (III)
dans laquelle:
$R_1$ a la même signification que dans la formule I, et avec l'ammoniac en solution acide;
ou:
b) que l'on fait réagir une α-cetoxime de la formule IIa:

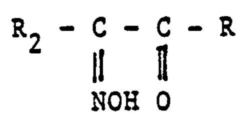

(IIa)

dans laquelle:
$R_2$ et R ont la même signification que dans la formule I, avec un aldéhyde de la formule III et l'ammoniac, pour obtenir un composé de la formule Ia:

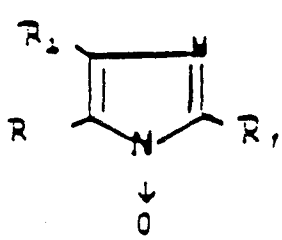

(Ia)

et que l'on réduit ce composé de manière en elle-même connue;
ou,
c) que l'on fait réagir en milieu alcalin un bromure d'acyle de la formule IV:

$R_2$-CO-CHR Br (IV),
dans laquelle:
$R_2$ et R ont la même signification que dans la formule I, sur une amidine de la formule V:

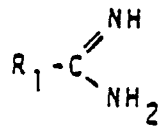

(V)

dans laquelle:
$R_1$ a la même signification que dans la formule I,
et qu'ensuite, si nécessaire, on convertit les composés obtenus en composés de la formule I, et que l'on convertit des bases imidazole correspondantes en sels, ou les sels en bases libres.

3. Utilisation de dérivés d'imidazole selon la revendication 1 comme indicateurs redox.

4. Indicateur redox selon la revendication 3, pour le décèlement de peroxyde d'hydrogène ou de substances ayant une action de peroxydase.

5. Réactif pour le décèlement de peroxyde d'hydrogène ou de substances ayant une action de peroxydase, formé d'un composé de la formule I et de substances actives et adjuvants usuels.

6. Réactif selon la revendication 5, caractérisé par le fait qu'on l'utilise sous forme de comprimés, lyophilisats, supports de réactif imprégnés ou pellicules de réactif.

Abb.1

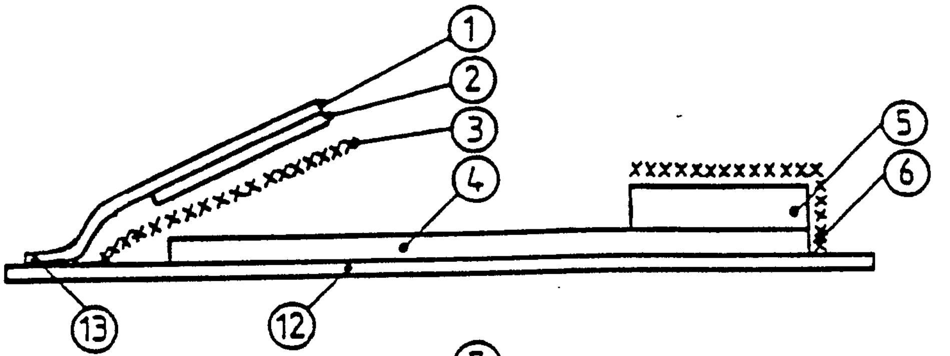


Abb.2

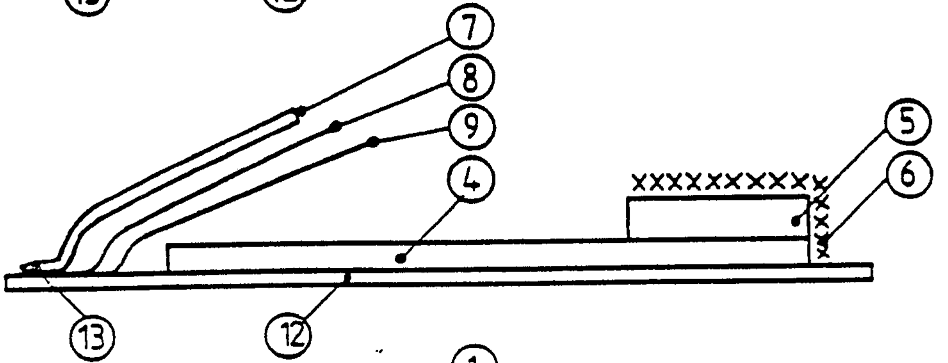


Abb. 3

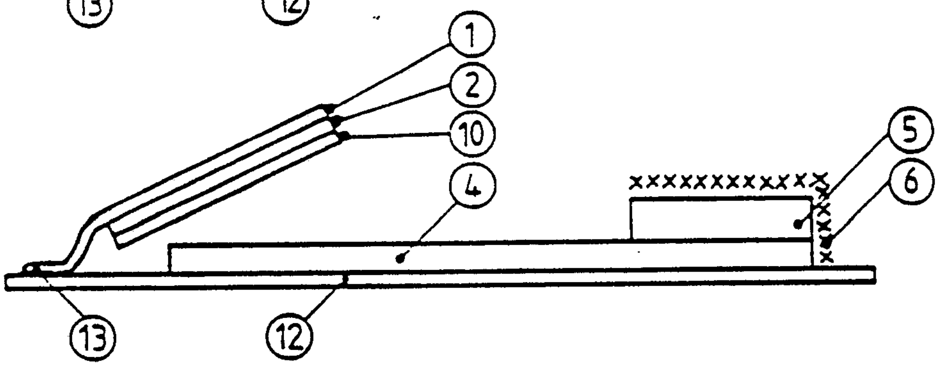


Abb.4